Europäisches Patentamt

European Patent Office

Office européen des brevets

(19)

(11) Veröffentlichungsnummer: **0 199 291**
**A1**

(12) # EUROPÄISCHE PATENTANMELDUNG

(21) Anmeldenummer: **86105318.9**

(22) Anmeldetag: **17.04.86**

(51) Int. Cl.⁴: **A 61 K 31/195**

(30) Priorität: **19.04.85 DE 3514328**

(43) Veröffentlichungstag der Anmeldung:
**29.10.86 Patentblatt 86/44**

(84) Benannte Vertragsstaaten:
**AT BE CH FR GB IT LI LU NL SE**

(71) Anmelder: **Deutsches Krebsforschungszentrum**
**Stiftung des öffentlichenRechts**
**Im Neuenheimer Feld 280**
**D-6900 Heidelberg 1(DE)**

(72) Erfinder: **Dröge, Wulf, Prof. Dr.**
**Mittlerer Rainweg 2/1**
**D-6900 Heidelberg(DE)**

(74) Vertreter: **Patentanwälte Müller-Boré, Deufel, Schön,**
**Hertel, Lewald, Otto**
**Postfach 26 02 47 Isartorplatz 6**
**D-8000 München 26(DE)**

(54) **Verwendung von L-Ornithin zur Herstellung von Mitteln zur selektiven Hemmung von T-Zell vermittelten Immunreaktionen.**

(57) Die Erfindung betrifft die Verwendung von L-Ornithin zur Herstellung von Mitteln zur selektiven Hemmung von T-Zell vermittelten Immunreaktionen, insbesondere zur selektiven Hemmung der Aktivierung von cytotoxischen T-Lymphozyteneffektorzellen.

EP 0 199 291 A1

Ein Hauptproblem bei der Organtransplantation ist das Auftreten von Immunabstoßungsreaktionen. Es besteht daher ein starker Bedarf nach Mitteln, welche diese Immunreaktionen unterdrücken. Eine Zusammenfassung findet sich in Transplantation Proc. Vol. XVII, Nr. 1 und 2 (Feb.1985). Diese Publikation zeigt einen Überblick über die bis jetzt bekannten und in der Klinik verwendeten Mittel, insbesondere auch Cyclosporin A, das sich als das wirksamste Mittel erwiesen hat.

Es wurde nun gefunden, daß L-Ornithin eine starke immunosuppressive Wirkung auf die Aktivierung cytotoxischer T-Lymphozyten (CTL) in vivo und in vitro hat, also die selektive Hemmung der Aktivierung von cytotoxischen T-Lymphozyten Effektorzellen bewirkt.

Dies ist überraschend, da diese Hemmwirkung bei den strukturanalogen L-Lysin und Putrescin oder bei den Aminosäuren L-Histidin und L-Alanin nicht zu beobachten ist.

Aus der US-PS 43 20 146 und zahlreichen Literaturstellen ist es bekannt, daß Ornithin und Arginin Schutz gegen die toxischen Effekte von Ammoniumsalzen bieten, jedoch ist die Gabe von Ornithin und Arginin als solche bei Patienten wegen deren verminderten Toleranz für Stickstoff beschränkt. Diese Verwendung für Hyperammonämie läßt aber keinen Schluß auf eine selektive Hemmung bestimmter Immunreaktionen zu.

Die immunhemmende Wirkung scheint selektiv auf die Aktivierung der CTL Precursorzellen ausgeübt zu werden, da L-Ornithin bei den für die Immunhemmung brauchbaren Konzentrationen keine Wirkung auf viele Typen proliferativer Reaktionen, einschließlich der allogenen mixed lymphocyte Reaktion, der Il-2-abhängigen proliferativen Antwort von Con-A-aktivierten Thymocyten und der IL-2-abhängigen Proliferation des W-2 T-Zellklons hat und auch keine Wirkung auf die Erzeugung von Interleukin 2 oder -Interferon durch Con-A-aktivierte C3H-Milzzellen oder PMA-aktivierte IL-4-Thymomatzellen.

0199291

Folglich ist der immunsuppressive Effekt von L-Ornithin selektiver als der von Cyclosporin A, von dem man weiß, daß es nicht nur die Aktivierung von cytotoxischer Aktivität sondern von proliferativen Reaktionen und die Produktion der Lymphokine IL-2 und $\gamma$-Interferon hemmt und unterdrückt. Demnach haben L-ornithinhaltige Mittel Vorteile gegenüber Cyclosporin A, das nicht nur der Ausbildung der cytotoxischen Effektorfunktion sondern auch die proliferative Antwort des T-Cell-Systems und die Induktion der Ornithindecarboxylase-Aktivität hemmt.

Ohne an eine Theorie gebunden zu sein könnte folgendes möglich sein. Es ist bekannt, daß durch Antigene oder Mitogene aktivierte Lymphozyten erhöhte intrazelluläre Spiegel von Ornithindecarboxylase ausdrücken, ein Schlüsselenzym für die Biosynthese von Polyaminen, die für Zellwachstum und Differenzierung erforderlich sind. Daher könnte der äußerliche Zusatz von L-Ornithin zu einem Überschuß an Polyaminen führen oder eine feed-back-Hemmung der ornithinerzeugenden Enzyme (d.h. Arginase) induzieren und hierdurch ein Ornithinmangel in der Spätphase der Immunantwort geschaffen werden.

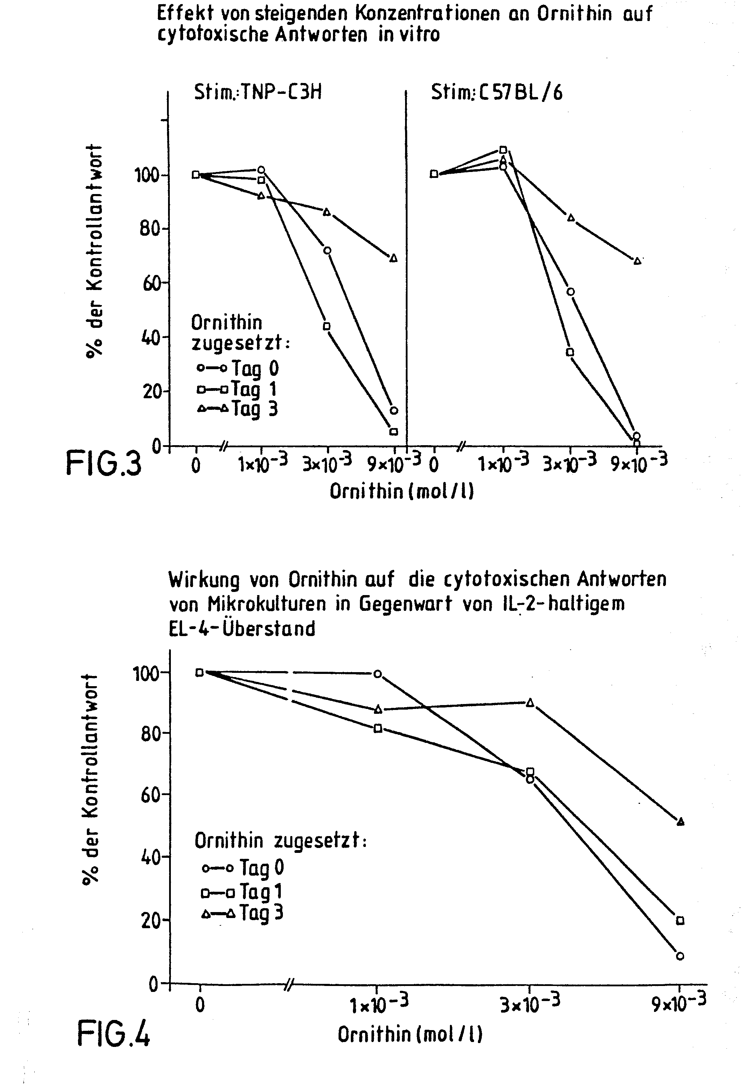

Die anzuwendenden Mengen betragen, wie aus der Figur 2 ersichtlich ist, etwa 0,1 bis 1 g/kg bei einer Durchschnittsdosis von etwa 0,1 bis 0,2 g/kg, wobei natürlich Abweichungen nach unten und oben möglich sind. Wegen der fehlenden Nebenwirkungen ist die Dosierung von Ornithin weniger kritisch als die von Cyclosporin A. Die Konzentration von Injektionslösungen kann zwischen 0,1 u. 1 molar sein. Als Träger für die anzuwendenden Präparate eignet sich z.B. physiologische Kochsalzlösung, mit welcher in üblicher Weise sterile Injektionsampullen zubereitet werden können. Das Mittel kann aber auch in Form von oralen Präparaten, z.B. Tabletten oder Kapseln konfektioniert werden, wobei die üblichen, bekannten Hilfsstoffe

0199291

für solche Präparate mitverwendet werden können (siehe z.B. Ullmanns Enzyklopäide d. techn. Chemie, 4. Aufl., Bd. 18, S. 151 - 161).

Im folgenden werden die Arbeitsmethoden beschrieben, die für die Versuche angewandt wurden, wobei darauf hingewiesen sei, daß die Methodik selbst bekannt, also Inhalt publizierter Arbeiten ist.

## Material und Arbeitsmethoden

Als Versuchtiere für die in vivo-Versuche wurden Mäuse vom Bomholtgard, Ry, Dänemark, verwendet, und zwar in den meisten Versuchen 8 bis 12 Wochen alte Mäuse.

## Aktivierung von cytotoxischen Antworten in vivo

Mäuse wurden mit 3 mg Cyclophosphamid (Endoxan, Asta, Brackwede, Deutschland) in 0,15 ml physiologischer Kochsalzlösung i.p. zwei Tage vor der Immunisierung behandelt. Die Immunisierung gegen trinitrophenylierte syngeneische Zellen wurde vorgenommen, indem die Mäuse mit 6x0,01 ml 20%igem Trinitrochlorbenzol (TNCB) in Aceton auf den vier Fußsohlen und zwei abdominalen Flächen bestrichen wurden.

Die Immunisierung gegen allogene Zellen wurde durchgeführt, indem man $5 \times 10^{7}$ bestrahlte (1500 rad) allogene Milzzellen gleichmäßig in die vier Fußsohlen in einem Gesamtvolumen von 0,2 ml verteilte. Die angegebenen Mengen von L-Ornithin wurden ebenfalls in die vier Fußsohlen zum Zeitpunkt der Immunisierung injiziert. Fünf Tage nach der Immunisierung wurden die Mäuse getötet und die Zellen der inguinalen und axilären Lymphknoten wurden direkt in einem 4-stündigen 51Cr-Release assay auf ihre cytotoxische Aktivität an Con A-aktivierten Lymphoblasten als Targetzellen getestet.

-4-

Aktivierung von cytotoxischen Antworten in Makrokulturen

---

20 Millionen Responderzellen (üblicherweise C3H Milz-zellen)wurden mit $1 \times 10^6$ bestrahlten (1500 rad) allogenen oder trinitrophenylierten syngenen Stimulatorzellen in einem Gesamtvolumen von 4,5 ml Kulturmedium (RPMI 1640, GIBCO Medium supplementiert mit 10mM L-Gutamin (GIBCO), Streptomycin/Penicillin (GIBCO), Streptomycin/ Penicillin (GIBCO; 100 units/ml), 0,5 % HEPES (GIBCO), 10% fötalem Kälberserum (GIBCO) und $3 \times 10^{-5}$ Mol/l 2-Mercaptoäthanol) 5 Tage bei 37°C in 5% $CO_2$ incubiert. Responder- und Stimulatorzellen waren Milzzellen. Die Kulturen wurden nach 5 Tagen auf cytotoxische Aktivität gegen Con A-aktivierte allogene oder trinitrophenylierte syngene Milzlymphoblasten in einem 4-Stunden 51Cr-Release assay in bekannter Weise getestet.

Aktivierung von cytotoxischen Antworten in Gegenwart von IL-2-haltigen Zellüberständen in Mikro-kulturen.

---

C3H-Milzzellen ($5 \times 10^4$) wurden als Responderzellen in $3 \times 10^5$ trinitrophenylierten und bestrahlten (1500 rad) C3H-Milzzellen und den angegebenen Mengen von IL-2-haltigen EL-4-Überstand in 0,2 ml Mikrokulturen incubiert. Die cytotoxische Aktivität gegen $2 \times 10^4$ trinitrophenylierte Con-A-aktivierte C3H-Milzzellenblasten wurde nach 5 Tagen in einem 4-Stunden 51Cr-Release assay in bekannter Weise getestet.

## Assay der DNA-Synthese

Die DNA-Synthese in Mikrokulturen wurde durch Zugabe von 1μCi von 3H-Thymidin pro 0,2 ml Kultur bestimmt. Die Mikrokulturen wurden üblicherweise weitere 8 h inkubiert und dann mit einem SCATRON-Cell-Harvester (Flow Laboratories) geerntet und die Filter wurden in einem Flüssigkeitsscintillationszähler gezählt. Jeder Punkt wurde in Vierfachbestimmung gemessen, wenn nicht anders angegeben.

## Bestimmung der Interleukin-2-Aktivität

Die IL 2 Titer wurden mit der IL 2 abhängigen W-2 T-Zelllinie in bekannter Weise bestimmt.

## Assay für die Interferon-Aktivität

Interferon-Titrationen wurden unter Verwendung eines Mikrotiterassays mit L 929-Zellen (0,2 ml Medium (RPMI 1640 + 5% FKS) pro Loch) und Vesicular Stomatitis Virus (Indiana-Stamm) als provozierendem Virus durchgeführt. Eine Einheit entspricht der minimalen Menge Interferon, die 50% der Zellen Schutz verleiht. Eine Mikrotitereinheit pro 0,2 ml entspricht 2 NIH Referenzeinheiten pro ml. Alle Titer sind in Laboreinheiten ausgedrückt.

## Herstellung von IL 2-haltigen Überständen

Die Überstände werden aus einer IL-2 produzierenden EL-4 Thymom-Sublinie nach Induktion mit Phorbolmyristinacetat (PMA) in bekannter Weise erhalten. Es wurden $10^6$ EL-4 Zellen pro ml zusammen mit 10 ng/ml PMA (SIGMA) 48 h inkubiert. Die Überstände wurden gesammelt und bei -20°C gefroren gelagert.

Die folgenden Beispiele zeigen die Ergebnisse und erläutern die Erfindung.

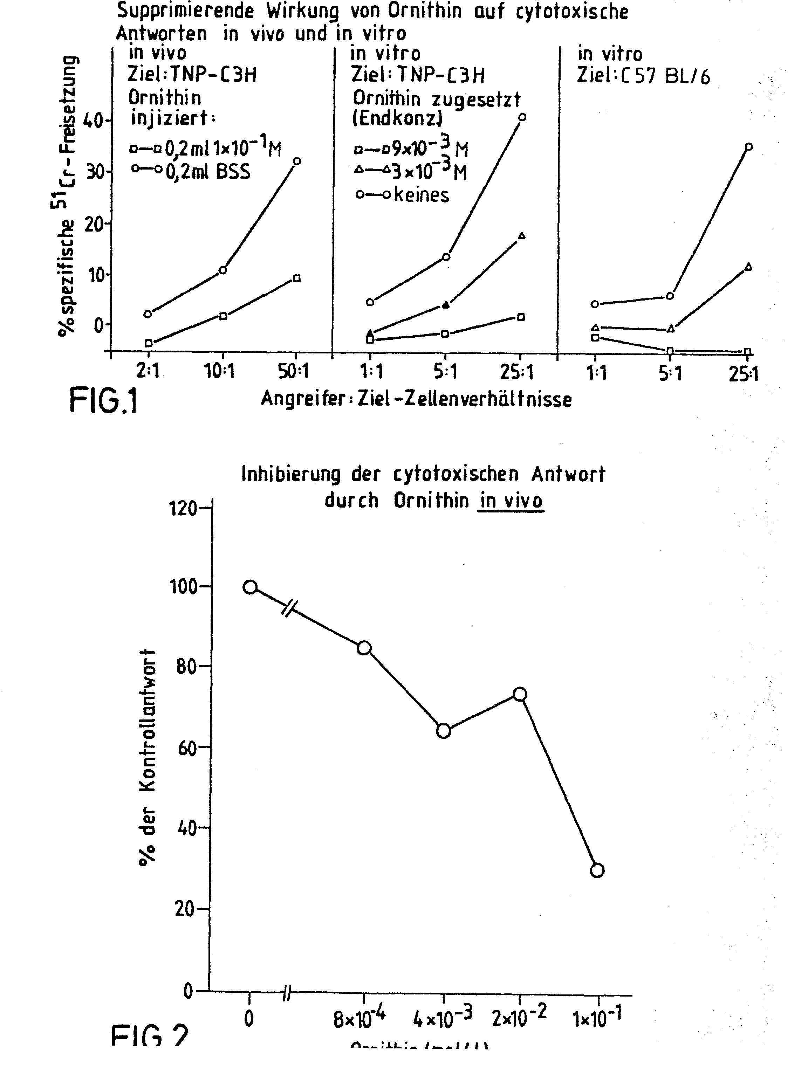

Beispiel 1: Supprimierende Wirkung   von Ornithin
auf cytotoxische Antworten in vivo und in vitro (Fig. 1)

3H Mäuse erhielten 3 mg Cyclophosphamid in 0,15 ml physiologischer Kochsalzlösung i.P. zwei Tage vor der Immunisierung. Die Immunisierung gegen tirinitrophenylierte syngeneische Zellen wurde wie im Abschnitt Arbeitsmethoden beschrieben, durchgeführt. Sofort nach der Immunisierung wurden 4x0,05 ml physiologische Kochsalzlösung mit den in Fig. 1 angegebenen Konzentrationen von Ornithin in die vier Fußsohlen injiziert. Die Mäuse wurden fünf Tage später getötet und die cytotoxische Aktivität der gepoolten inguinalen und axillären Lymphknotenzellen gegen 51Cr-markierte trinitrophenylierte C3H Targetzellen wurde bei dem angegebenen Angreifer: Ziel-Zellen-Verhältnis getestet.

Der Effekt in vitro (mittlere und rechte Zeichnung der Fig. 1) wurde wie folgt bestimmt:
C3H Milzzellen ($2 \times 10^7$) wurden mit $1 \times 10^6$ bestrahlten und Trinitrophenylen-behandelten C3H Milzzellen (Mitte) oder $1 \times 10^6$ bestrahlte C57BL/6 Milzzellen (rechts) und mit den angegebenen Endkonzentrationen von Ornithin in 4,5 ml Kulturen 5 Tage inkubiert. Die cytotoxische Aktivität gegen die entsprechenden Targetzellen wurde nach 5 Tagen getestet.

Beispiel 2 :     Effekt von steigenden Dosen von L-Ornithin auf die cytotoxische Antwort in vivo (Fig. 2)


C3H Mäuse wurden mit Cyclophosphamid behandelt und mit Trinitrochlorbenzol immunisiert, wie in Beispiel 1
beschrieben. Sofort nach der Immunisierung wurden 4 x
0,05 ml BSS mit steigenden Konzentrationen von Ornithin
in die vier Fußflächen injiziert. Nach 5 Tagen wurde
die cytotoxische Aktivität der gepoolten inguinellen und
axillären Lymphknotenzellen gegen Trinitrophenylenbehandelte C3H Targetzellen bei Angreifer: Ziel-Zellen-
Verhältnissen von 50:1, 10:1 und 2:1 getestet.
Figur 2    zeigt die relative cytotoxische Aktivität
beim Angreifer:Ziel-Zellen- Verhältnis von 50:1 (die
Kontrollantwort ohne Ornithin zeigte 32% der spezifischen
$^{51}$Cr-release).


Beispiel 3:     Effekt von steigenden Dosen Ornithin
auf die cytotoxische Antwort in vitro (Fig. 3)


C3H Milzzellen (2 x 10$^7$) wurden mit 1 x 10$^6$ bestrahlten
und Trinitrophenylen-behandelten C3H Milzzellen (links)
oder 1 x 10$^6$ bestrahlten C57BL/6 Milzzellen (rechts)
in 4,5 ml Kulturen 5 Tage inkubiert. Am Tag 0, Tag 1 oder
Tag 3 wurde den Kulturen 0,2 ml Kulturmedium mit
steigenden Konzentrationen von L-Ornithin zugesetzt.
Die Abszisse zeigt die Endkonzentration von Ornithin
in der Kultur. Die cytotoxische Aktivität gegen die entsprechenden Targetzellen wurde nach 5 Tagen bei Angreifer:
Ziel-Zellen-Verhältnissen von 25:1, 5:1 und 1:1 untersucht.    Figur 3 zeigt    die relative cytotoxische
Aktivität beim Angreifer: Ziel-Zellen-Verhältnis 25:1.
(Die Kontrollantwort ohne Ornithin zeigte 54% (links)
bzw. 50% (rechts) $^{51}$Cr-release).

Beispiel 4:        Wirkung von Ornithin auf die cytotoxischen Antworten von Mikrokulturen in Gegenwart von
IL-2-haltigem EL-4 Überstand   (Fig. 4)

C3H Milzzellen (5 x $10^4$) wurden mit 3 x $10^5$ bestrahlten
(1500 rad) Trinitrophenylen-behandelten C3H Milzzellen und
0,015 ml EL-4 Überstand (2 units IL-2) inkubiert. Die
Kulturen wurden am Tag 0, Tag 1 oder Tag 3 mit 0,02 ml
Kulturmedium mit steigenden Konzentrationen von L-
Ornithin  ergänzt. Die Abszisse von Fig. 4 gibt die Endkonzentration von Ornithin in den Kulturen an. Die cytotoxische
Aktivität gegen TNP-Hapten-behandelte syngene Targetzellen
(2 x $10^4$ pro Loch) wurde am Tag 5 getestet. (Die Kontrollantwort zeigte 54% bei $^{51}$Cr-Freisetzung)

Es zeigt sich somit, daß Ornithin eine starke suppressive
Wirkung auf in vivo- und in vitro-cytotoxische Antworten
gegen haptenierte syngene und allogene Targetzellen ausübt (Fig. 1). Die cytotoxische Antwort von Cyclophosphamid
behandelten C3H Mäusen gegen allogene C57BL/6 Zellen
in vivo war immer, von vornherein, bereits relativ schwach,
wurde jedoch ebenfalls durch Injektion von L-ornithine
supprimiert. Die Dosiswirkungskurven für die Effekte von Ornithin in vivo und in vitro
sind in den Figuren 2 bzw. 3 gezeigt. Die Figur 3 zeigt
auch, daß die Suppression ausgeprägter ist, wenn Ornithin
in der frühen Phase, d.h. am Tag 0 oder Tag 1, zugesetzt
wird, als am Tag 3 der cytotoxischen Antwort. Der
suppressive Effekt wird nicht mit den Strukturanalogen
L-Lysin und Putrescin oder mit den Aminosäuren L-Histidin
und L-Alanin beobachtet, wie Tabelle I zeigt.

TABELLE I

Effekt von verschiedenen Aminosäuren und Putrescin auf die Aktivierung von cytotoxischen T-Lymphocyten

Stimulans und Target

| | TNP-C3H | | | C57BL/6 | | |
| | 25:1 | 5:1 | 1:1 | 25:1 | 5:1 | 1:1 |
|---|---|---|---|---|---|---|
| Keine | 54,9 | 32,0 | 16,3 | 33,5 | 17,7 | 4,8 |
| L-Ornithin | 7,9 | 3,7 | 3,2 | 1,8 | -1,7 | -0,2 |
| L-Lysin | 60,7 | 39,9 | 21,5 | 41,7 | 19,1 | 9,0 |
| L-Hystidin | 48,5 | 27,8 | 11,1 | 24,5 | 11,4 | 5,1 |
| L-Alanin | 40,5 | 25,3 | 9,8 | 19,2 | 5,3 | 6,7 |
| Putrescin | 44,2 | 24,8 | 9,6 | 30,2 | 11,9 | 7,2 |

C3H Milzzellen ($2 \times 10^7$) wurden mit $5 \times 10^6$ bestrahlten trinitrophenylierten C3H Milzzellen oder C57BL/6 Milzzellen in 5 ml der Kulturen inkubiert, die zusätzlich zu den Standardmedienbestandteilen die angegebenen Aminosäuren oder Putrescin in Konzentrationen von $1 \times 10^{-2}$ mol/l enthielten. Die Ergebnisse geben die cytotoxische Aktivität (% $^{51}$Cr-Freisetzung) gegen homologe Targetzellen nach 5 Tagen bei Angreifer:Targetzellen-Verhältnissen von 25:1, 5:1 und 1:1 an.

Um zu unterscheiden, ob Ornithin die Aktivierung von CTL-Precursorzellen direkt oder nur indirekt durch die Hemmung von Helper-T-Zellen hemmt, wurden die Experimente in IL-2-haltigen Mikrokulturen durchgeführt. Die Ergebnisse dieser Beispiele (Fig. 4) machten offenbar, daß Ornithin einen starken suppressiven Effekt auch auf cytotoxische Antworten in IL-2-haltigen Kulturen ausübt. Die Suppressionswirkung war wiederum ausgeprägter, wenn Ornithin am Tag 0 oder Tag 1 zugesetzt wurde, als wenn es am Tag 3 der Kultur geschah.

Die Applikation des ornithinhaltigen Mittels wird somit zweckmäßig so erfolgen, daß der gewünschte Ornithinspiegel am Tage Null, also bei der Operation, zur Verfügung steht.

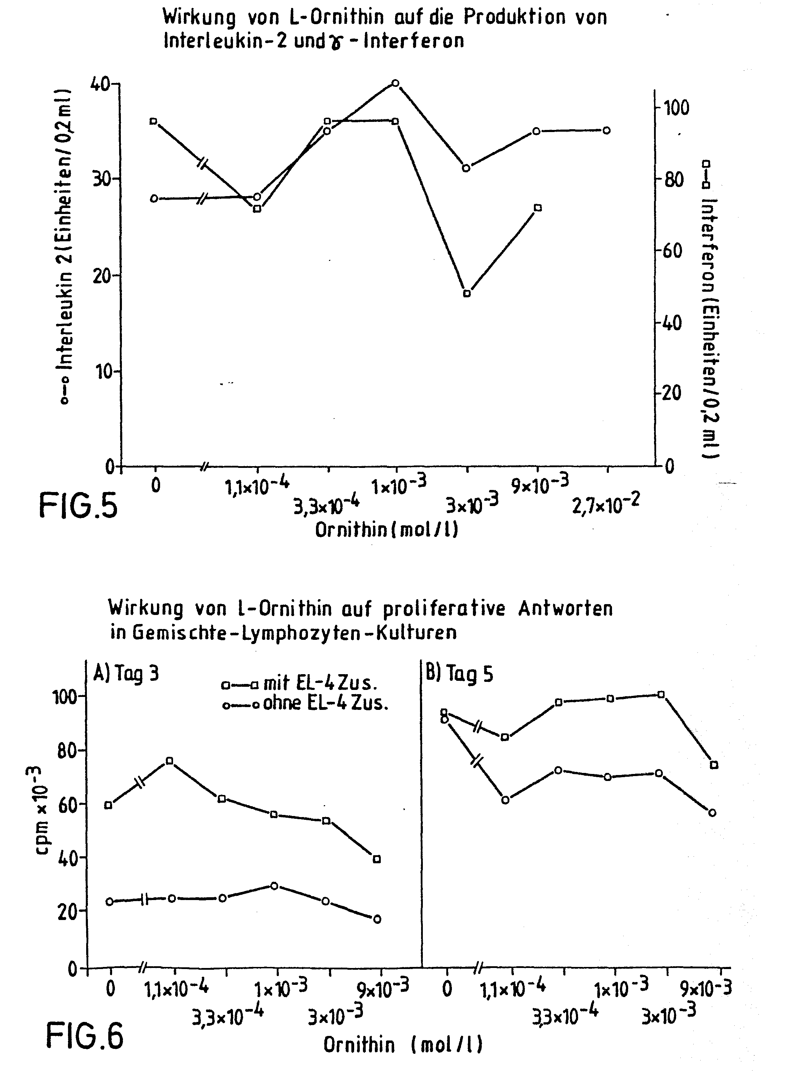

Beispiel 5:        Wirkung von L-Ornithin auf die Produktion von Interleukin-2 und $\chi$-Interferon (Fig. 5)

C3H Milzzellen (1 x $10^7$/ml) wurden mit Conacanavalin A (5 µg/ml) und den angegebenen Endkonzentrationen von L-Ornithin für 24 h in 4 ml Makrokulturen inkubiert. Die resultierenden Überstände wurden dann durch Zentrifugation isoliert und auf Interleukin-2 und Interferon-Aktivität getestet, wie im Methodenteil beschrieben. Die Interleukin-2-Produktion von PMA-aktivierten EL-4 Thymomzellen wurde ebenfalls untersucht und war durch diese Konzentrationen von L-Ornithin ebenfalls nicht supprimiert.

Die Konzentration von $9 \times 10^{-3}$ mol/l L-Ornithin, von der gefunden wurde, daß sie die cytotoxischen Antworten vollständig supprimiert,hat also keinen wesentlichen Effekt auf die Produktion von IL-2 und IFN-$\gamma$ (Fig. 5). Ansteigende Konzentrationen von Ornithin wurden Kulturen von con-A-aktivierten Konzentrationen von Ornithin C3H Milzzellen ($10^7$ Zellen + 5 µg con-A pro ml) zugesetzt. Die erhaltenen Überstände wurden 24 h später isoliert und auf Interleukin-2 und Interferonaktivität getestet. Die Ergebnisse (Fig. 5) zeigen, daß die Produktion beider Lymphokin-Aktivitäten nicht merklich durch den Zusatz von Ornithin beeinträchtigt wurde. Ähnliche Ergebnisse wurden bezüglich der IL-2 und IFN-$\gamma$ -Titer nach 48 h Kultur und außerdem auch bezüglich der IL-2-Titer in ornithinhaltigen Kulturen von Phorbolmyristinacetat aktivierten EL-4 Thymomazellen gefunden. C3H Milzzellkulturen mit 1 µg/ml con-A sowie allogene "Gemischte-Lymphozyten-Kulturen" mit $10^7$ nicht-bestrahlten C3H Milzzellen plus $10^7$ nicht-bestrahlten C57BL/6 Milzzellen wurden ebenfalls mit steigenden Konzentrationen von Ornithin angesetzt. Ihre Überstände zeigten jedoch unabhängig von der Menge des zugesetzten Ornithins keine IL-2 oder Interferon-Aktivität . Kontrollösungen, die steigende Mengen von Ornithin im Kulturmedium enthielten, zeigten ebenfalls keine Lymphokin-Aktivitäten in den beiden Assay-Systemen . Diese Kontrollversuche zeigten, daß Ornithin selbst keine Interleukin-2 oder Interferon-Aktivität aufweist und die Lymphokin-Produktion in Lymphozyten-Kulturen nicht induziert. Außerdem hat Ornithin keinen verstärkenden oder hemmenden Effekt auf die beiden Assay-Systeme.

0199291

Beispiel 6 : Wirkung von Ornithin auf proliferative Antworten

Das folgende Beispiel zeigt die Wirkung von Ornithin auf drei Typen von proliferativen T-Zellantworten. Zunächst wurden "Gemischte-Lymphozyten-Kulturen" mit $3 \times 10^5$ CH3 Milzzellen und $3 \times 10^5$ bestrahlten(1500 rad) C57BL/6 Milzzellen als Stimulatorzellen mit oder ohne Zusatz von 0,15 ml EL-4-Überstand (2 units IL-2 pro Kultur) mit steigenden Dosen von L-Ornithin inkubiert. Die Ergebnisse (Fig. 6) zeigen, daß Ornithin bei allen getesteten Konzentrationen keine Wirkung auf die Antwort der DNA-Synthese ($^3$H-Thymidin-Einbau) zeigt.

Fig. 6 zeigt die Wirkung von L-Ornithin auf die proliferative Antwort in der allogenen mixed Lymphozytkultur (LMC), wobei C3H Milzzellen ($3 \times 10^5$) mit $3 \times 10^5$ bestrahlten (1500 rad) C57BL/6 Milzzellen mit oder ohne Zusatz von 0,015 ml EL-4 Überstand in 0,2 ml Mikrokulturen inkubiert wurden. Die DNA-Synthese wurde nach 3 und 5 Tagen bestimmt, wie im Methodenteil beschrieben.

Beispiel 7

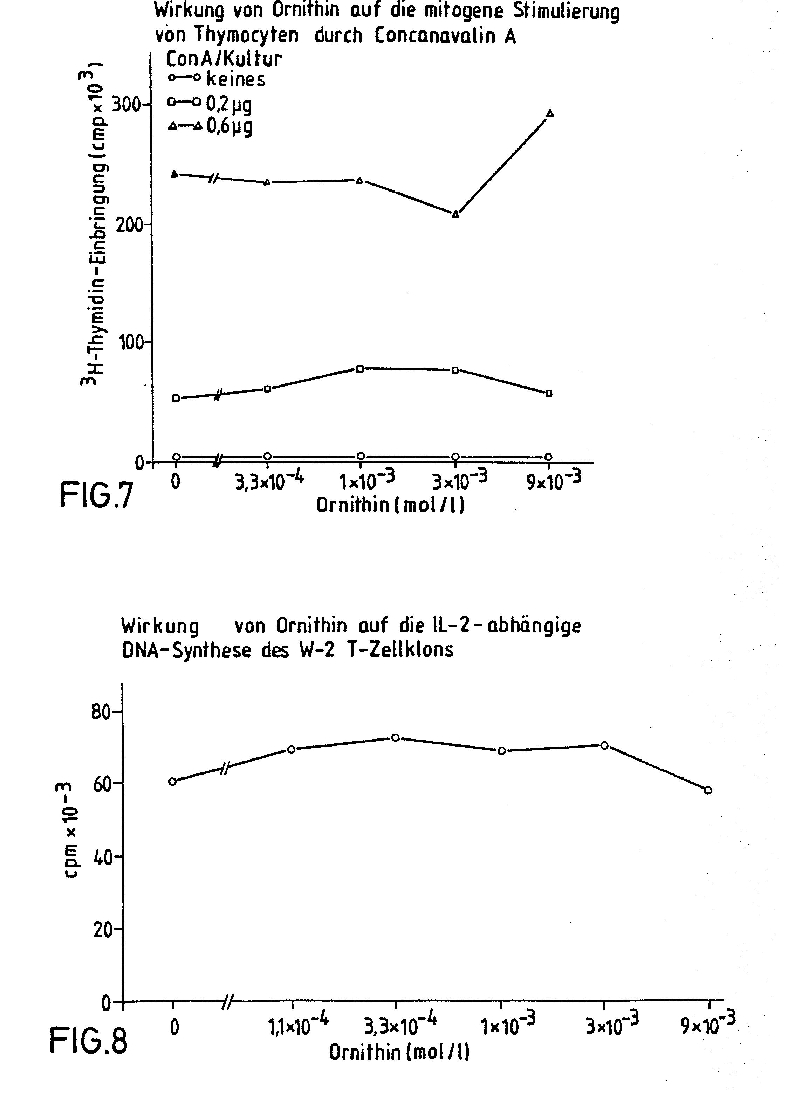

In einem weiteren Beispiel wurden C3H Thymocyten (1 x $10^5$ Zellen)mit IL-2 (2 units pro Kultur) und steigenden Konzentrationen von Concanavalin A und L-Ornithin in 0,2 ml Mikrokulturen inkubiert. Die Ergebnisse (Fig. 7) zeigen wiederum, daß Ornithin keinen Effekt auf die DNA-Synthese der reagierenden Lymphozyten zeigte.

Die Fig. 7 zeigt die Wirkung von Ornithin auf die Mitogenstimulierung von Thymocyten durch Concanavalin A, wobei C3H Thymuszellen (1 x $10^5$) mit den angegebenen Mengen von Concanavalin A und mit 2 Einheiten IL-2 und den angegebenen Konzentrationen von L-Ornithin in 0,2 ml

-13-

Mikrokulturen inkubiert wurden. Die DNA Synthese wurde nach 3 Tagen, wie im Methodenteil beschrieben, getestet.

Beispiel 8

In diesem Beispiel wurden $10^4$ Zellen des W-2 T-Zellklons mit Interleukin-2 (0,5 units pro Kultur) und steigenden Dosen von L-Ornithin in 0,2 ml Mikrokulturen 20 h bei 37°C inkubiert. Die W-2-Zellen wurden dann für die DNA-Synthese untersucht, wie für den Interleukin-2 Assay beschrieben (Methodenabschnitt). Wiederum hatte Ornithin keine Wirkung auf die DNA-Synthese dieses T-Zellklons.

Figur 8 zeigt die Wirkung von L-Ornithin auf die IL-2-abhängige DNA-Synthese des T-Zellklons W-2, wobei Zellen des T-Zellklons W-2 ($10^4$) mit Interleukin-2 (0,5 Einheiten) und steigenden Konzentrationen von L-Ornithin in 0,2 ml Mikrokulturen für 20 h inkubiert wurden. Die Kulturen wurden dann auf ihre DNA-Synthese untersucht, wie im Interleukin-2 Assay im Methodenteil beschrieben.

Die Beispiele zeigen somit, daß L-Ornithin eine starke immunosuppressive Wirkung auf die Aktivierung cytotoxischer T-Lymphozyten in vivo und in vitro hat. Andererseits hat Ornithin in ähnlichen Konzentrationen keine Wirkung auf die Erzeugung von Interleukin-2 oder $\gamma$-Interferon durch con-A-aktivierte C3H Milzzellen oder PMA-aktivierte EL-4 Thymomazellen. Es hat weiterhin keine Wirkung auf viele Typen proliferativer Reaktionen, einschließlich der allogenen "Gemischten-Lymphozyten-Reaktion", die IL-2-abhängige proliferative Antwort von con-A-aktivierten Thymocyten und die IL-2-abhängige Proliferation des W-2 T-Zellklons. Dies weist darauf hin, daß L-Ornithin bei den benutzten Konzentrationen keinen allgemeinen toxischen Effekt auf die reagierenden Zell-

C199291

populationen hat, sondern einen selektiven immunsuppressiven Effekt auf die Aktivierung der CTL-Precursorzellen ausübt. Dies wird außerdem durch die Tatsache gestützt, daß die cytotoxischen Antworten stark supprimiert wurden, wenn Ornithin während der ersten zwei Tage der Reaktion zugesetzt wurde, jedoch wesentlich weniger gehemmt waren, wenn Ornithin am Tag 3 zugesetzt wurde. L-Ornithin scheint somit die zelluläre Immunantwort auszuschalten, ohne die unerwünschten Nebenwirkungen von Cyclosporin A zu zeigen.

K 1712

## Patentansprüche

1. Verwendung von L-Ornithin für die Herstellung von Mitteln zur selektiven Hemmung von T-Zell vermittelten Immunreaktionen.

2. Verwendung nach Anspruch 1 zur Herstellung von Mitteln zur selektiven Hemmung der Aktivierung von cytotoxischen T-Lymphozyteneffektorzellen.

3. Verwendung nach Anspruch 1 oder 2, dadurch gekennzeichnet, daß L-Ornithin in einer Menge von 0,1 bis 1 g/kg Körpergewicht eingesetzt wird.

4. Mittel zur selektiven Hemmung von T-Zell vermittelten Immunreaktionen, insbesondere zur selektiven Hemmung der Aktivierung von cytotoxischen T-Lymphozyteneffektorzellen bestehend aus L-Ornithin, gelöst in physiologischer Kochsalzlösung oder pyrogenfreiem Wasser oder in Form von üblichen Oralpräparaten mit üblichen Hilfsstoffen.

5. Mittel nach Anspruch 4, dadurch gekennzeichnet, daß L-Ornithin in einer Konzentration von 0,1 bis 1 mol/l Trägerflüssigkeit vorliegt.

6. Verfahren zur selektiven Hemmung von T-Zell vermittelten Immunreaktionen, insbesondere zur selektiven Hemmung der Aktivierung von cytotoxischen T-Lymphozyteneffektorzellen, dadurch gekennzeichnet, daß man Ornithin, gelöst in physiologischer Kochsalzlösung oder pyrogenfreiem Wasser oder als Oralpräparat, insbesondere Tabletten oder Kapseln, in einer pysiologisch wirksamen Menge oral oder parenteral verabreicht.

7. Verfahren nach Anspruch 6, dadurch gekennzeichnet, daß L-Ornithin in einer Menge von 0,1 bis 1 g/kg Körpergewicht eingesetzt wird.

8. Verfahren nach Anspruch 6, dadurch gekennzeichnet, daß L-Ornithin in einer Konzentration von 0,1 bis 1 mol/l Trägerflüssigkeit eingesetzt wird.

0199291

Supprimierende Wirkung von Ornithin auf cytotoxische
Antworten in vivo und in vitro

**FIG.1**

Inhibierung der cytotoxischen Antwort
durch Ornithin _in vivo_

FIG 2

Effekt von steigenden Konzentrationen an Ornithin auf
cytotoxische Antworten in vitro

Stim.:TNP-C3H          Stim.:C57BL/6

% der Kontrollantwort

Ornithin
zugesetzt:
o—o Tag 0
□—□ Tag 1
△—△ Tag 3

Ornithin (mol/l)

**FIG.3**

Wirkung von Ornithin auf die cytotoxischen Antworten
von Mikrokulturen in Gegenwart von IL-2-haltigem
EL-4-Überstand

% der Kontrollantwort

Ornithin zugesetzt:
o—o Tag 0
□—□ Tag 1
△—△ Tag 3

Ornithin (mol/l)

**FIG.4**

Wirkung von L-Ornithin auf die Produktion von
Interleukin-2 und γ - Interferon

FIG.5

Wirkung von L-Ornithin auf proliferative Antworten
in Gemischte-Lymphozyten-Kulturen

FIG.6

Wirkung von Ornithin auf die mitogene Stimulierung
von Thymocyten durch Concanavalin A

FIG.7

Wirkung von Ornithin auf die IL-2-abhängige
DNA-Synthese des W-2 T-Zellklons

FIG.8

**0199291**

Nummer der Anmeldung

EUROPÄISCHER TEILRECHERCHENBERICHT,
der nach Regel 45 des Europäischen Patentübereinkommens für das weitere Verfahren als
europäischer Recherchenbericht gilt

Europäisches
Patentamt

EP 86 10 5318

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | JOURNAL OF IMMUNOLOGY; Band 130, Nr. 4, April 1983, Seiten 1937-1942; The American Association of Immunologists, US M. DY et al.: "Evidence for a lymphokine enhancing arginase activity during allograft rejection."<br><br>* Insgesamt *<br><br>--- | <br><br><br><br><br><br>1-5 | A 61 K 31/195 |
| Y | JOURNAL OF IMMUNOLOGY, Band 123, Nr. 2, August 1979, Seiten 817-824; The Williams & Wilkins Co., US G.R. KLIMPEL et al.: "Cyclic AMP-dependent protein kinase activation and the induction of ornithine decarboxylase during lymphocyte mitogenesis."<br><br>* Insgesamt *<br><br>--- | <br><br><br><br><br><br><br>1-5 | RECHERCHIERTE SACHGEBIETE (Int. Cl 4) |
| Y | EUR. J. IMMUNOL., Band 13, 1983, Seiten 977-983; Verlag Chemie GmbH, | | A 61 K 31/00 |

## UNVOLLSTÄNDIGE RECHERCHE ./.

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5

Unvollständig recherchierte Patentansprüche:

Nicht recherchierte Patentansprüche: 6-8

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 10-07-1986 | THEUNS |

**0199291**

Nummer der Anmeldung

Europäisches
Patentamt

**EUROPÄISCHER TEILRECHERCHENBERICHT**  EP  86 10 5318

− 2 −

| | **EINSCHLÄGIGE DOKUMENTE** | | **KLASSIFIKATION DER ANMELDUNG (Int. Cl. 4)** |
|---|---|---|---|
| Kategorie | Kennzeichnung des Dokuments mit Angabe, soweit erforderlich, der maßgeblichen Teile | betrifft Anspruch | |
| | Weinheim, DE<br>E. SCHNEIDER et al.: "Demonstration of a concanavalin A-induced lympho-kine enhancing arginase activity. Effect of presensitization by a skin allograft."<br><br>* Insgesamt * | 1-5 | |
| Y | ADVANCES IN CANCER RESEARCH, Band 36, Nr.1, 1982, Seiten 1-102; Academic Press Inc.<br>G. SCALABRINO et al.: "Polyamines in mammalian tumors. Part II'."<br><br>* Seite 7, letzter Absatz; Seite 8, Zeilen 1-3,10-13; Seite 62, Abschnitt IV, Absatz 2; Seite 63, Zeilen 1-4; Seite 66, Zeilen 4-9 * | 1-5 | **RECHERCHIERTE SACHGEBIETE (Int. Cl.4)** |
| X,P | JOURNAL OF IMMUNOLOGY, Band 134, Nr.5, Mai 1985, Seiten 3379-3383; W. DROGE et al.: "Suppression of cytotoxic T lymphocyte activation by L-ornithine."<br><br>* Insgesamt * | 1-5 | |
| X,P | CELLULAR IMMUNOLOGY, Band 92, Nr.2, Mai 1985, Seiten 359-365; Academic Press Inc.<br>R. JÄNICKE et al.: "Effect of L-or-nithine on proliferative and cyto-toxic T-cell responses in alloge-neic and syngeneic mixed leukocyte cultures."<br><br>* Insgesamt * | 1-5 | |
| X | PATH. BIOL., Band 31, Nr.3, 1983, Seiten 191-194; Paris, FR<br>J.L. PASQUALI et al.: "La stimula-tion lymphocytaire in vitro par le pockeweed mitogène chez les sujets normaux et les sujets dénutris. Influence des sels d'ornithine." | | |

./..

**Europäisches Patentamt**

## EUROPÄISCHER TEILRECHERCHENBERICHT

EP 86 10 5318

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| | * Zusammenfassung; Seite 194, rechte Spalte, letzter Absatz *  --- | 1-5 | |
| Y | C.R. ACAD. SC. PARIS, Band 289, Nr. 16, D, 17. Dezember 1979, Seiten 1245-1249; M. DY et al.: "Immunologie: Production d'urée au cours des cultures mixtes leucocytaires (MLC). Effet d'une présensibilisation par une allogreffe de peau (1)."  * Insgesamt *  --- | 1-5 | |
| X | THE MERCK INDEX, 10. Auflage, 1983, Seiten 985-986; Merck & Co., Inc., Rahway, N.J., US  * Ref. Nr. 6748: "Ornithine" *  -------- | 1-5 | RECHERCHIERTE SACHGEBIETE (Int. Cl. 4) |